# DEMANDE DE BREVET EUROPEEN

(11) **EP 1 297 806 A2**
(43) Date de publication de la demande: **02.04.2003**
(21) Numéro de dépôt: 02079053.1
(22) Date de dépôt: 24.09.2002
(51) Int. Cl.: A61F 13/471, A61F 13/505

(54) **Système de protection hygiénique pour incontinence urinaire masculine et accessoires destinés à un tel système**

(30) Priorité: 26.09.2001 BE 200100618
(71) Demandeur: Rixhon, Raoul, 7700 Mouscron (BE)
(72) Inventeur: Rixhon, Raoul, 7700 Mouscron (BE)
(74) Mandataire: Ostyn, Frans

(57) **Abrégé**

L'invention concerne un système de protection hygiénique pour incontinance urinaire masculine, impliquant
des pochettes en matière imperméable et
des pochettes ou fourreaux en matière absorbante, se glissant dans lesdites pochettes imperméables,
ainsi qu'un caleçon en matière textile quelconque, muni sur sa face interne au niveau de l'organe urinaire, d'une poche en matière textile quelconque, ladite poche en matière textile etant destinée à recevoir une desdites pochettes en matière imperméable munie d'une desdites pochettes ou fourreaux en matière absorbante.

L'invention concerne également des caleçons munis sur leur face interne au niveau de l'organe urinaire, d'une poche en matière textile quelconque, ainsi que des ensembles constitués d'une pochette en matière imperméable et d'une pochette ou fourreau en matière absorbante, destinés à être utilisé dans le système selon l'invention.

## Description

L'invention concerne un système de protection hygiénique pour incontinance urinaire masculine, impliquant une pièce de matière absorbante disposée dans une pièce de sous-vêtement destinée à être portée au niveau de l'organe urinaire masculin.

Les systèmes connus de ce type présentent divers inconvéniants.

Ainsi, les systèmes de type barboteuse en matière imperméable ont le désavantage d'être fort épais sur le devant, et de comporter des élasiques désagréables

Afin de remédier aux inconvéniants des divers systèmes connus, la présente invention procure un nouveau système de protection qui implique
des pochettes en matière imperméable et
des pochettes ou fourreaux en matière absorbante, se glissant dans lesdites pochettes imperméables, ainsi qu'
un caleçon en matière textile quelconque, muni sur sa face interne au niveau de l'organe urinaire, d'une poche en matière textile quelconque, ladite poche en matière textile etant destinée à recevoir une desdites pochettes en matière imperméable munie d'une desdites pochettes ou fourreaux en matière absorbante.

Dans le présent texte le mot caleçon s'entend à la fois comme sous-vêtement et comme vêtement de loisir (tel un short, bermuda, boxer, etc.)

Selon un mode de réalisation préféré de l'invention, les pochettes en matière imperméable et les pochettes ou fourreaux en matière absorbante sont de type jetable.

Le caleçon et la pochette interne sont de préférence constitués d'une matière textile identique ou permettant un même traitement au lavage.

L'ouverture de la poche disposée sur la face interne du calecon est de préférence située à environ 20 à 25 cm, plus particulièrement à environ 22 cm, de la taille d'un homme standard.

La poche peut, de manière standard, être disposée du coté gauche de la jambe, légèrement en oblique, sur la face interne du caleçon, car il a été constaté que la majorité des hommes "portent à gauche".

L'invention concerne également, comme nouvel article de commerce, un caleçon muni sur sa face interne au niveau de l'organe urinaire, d'une poche en matière textile quelconque.

Dans le caleçon selon l'invention l'ouverture de la poche, disposée sur la face interne du caleçon, est, de préférence, située à environ 20 à 25 cm, plus particulièrement à environ 22 cm, de la taille d'un homme standard.

La poche disposée sur la face interne du caleçon est de préférence située du coté gauche de la jambe, légèrement en oblique.

L'invention concerne, enfin, des ensembles constitués d'une pochette en matière imperméable et d'une pochette ou fourreau en matière absorbante, en tant qu'articles de commerce destinés à être utilisés dans le système selon l'invention.

L'invention est illustrée par un mode de réalisation concret, décrit- ci dessous en référence aux dessins annexés, dans lesquels
la figure 1 montre un caleçon selon l'invention,
la figure 2, illustre la position de la poche sur la face interne du caleçon,
la figure 3 montre une pochette ou foureau en matière absorbante, selon l'invention,
la figure 4 illustre l'utilisation de cette pochette ou foureau en matière absorbante,
la figure 5 et 6 montrent une pochette en matière imperméable, selon l'invention,
les figures 7 et 8 montrent un mode de réalisation de la poche en matière textile destinée à recevoir une pochette en matière imperméable munie d'une pochette ou fourreau en matière absorbante.

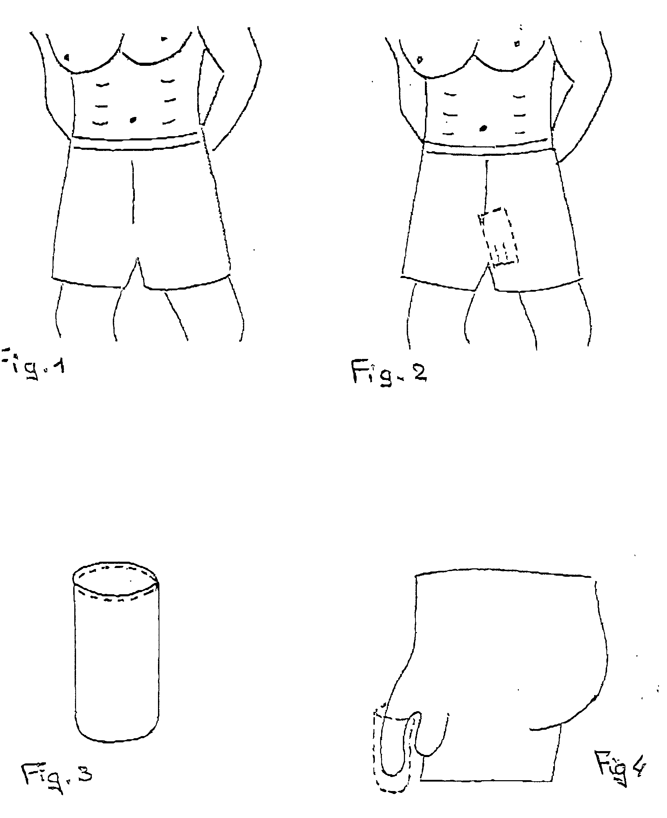

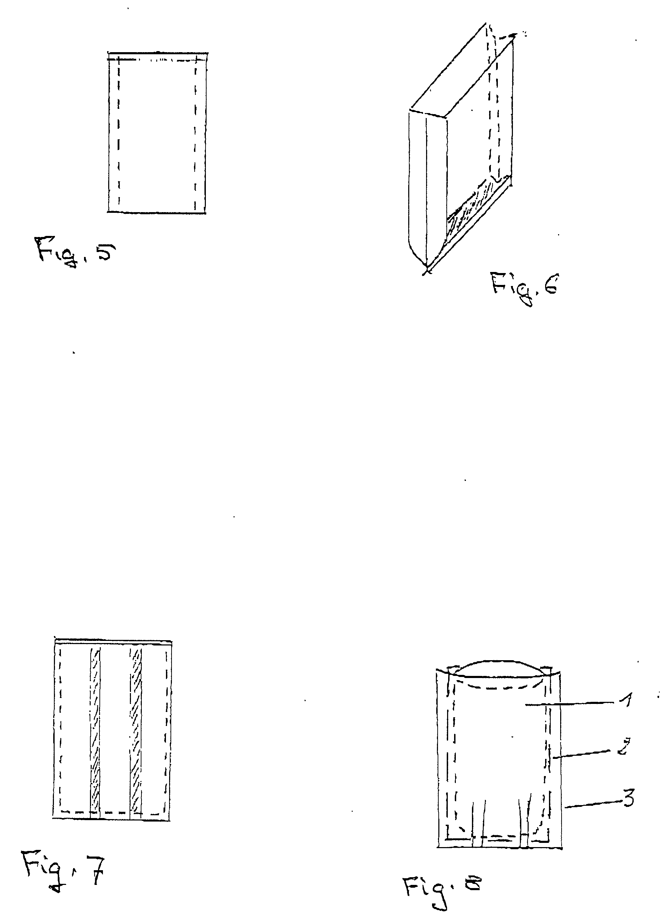

Le caleçon se porte en sous-vêtement ou à la plage. Modèle short, bermuda ou boxer convient à tout age.

Avantages : aisance et discrétion, hygiénique et pratique.

Un changement à faire ? Facile ! Il suffit de retirer la Housse plastique avec la protection absorbante, de fermer la housse et de jeter le tout dans la poubelle.

Vous pouvez facilement emporter une ou plusieurs protections absorbantes, Très discrètes dans leurs housses jetables. Vous ne devrez pas changer le caleçon car vous aurez une protection totale durant toute la journée.

La protection absorbante ( 1 ) selon la figure ( 3 ) constitue un modèle fermé, donc pas de fuite de côté. Il se place dans la housse jetable ( 2 ) selon la figure 5 - 6.

La housse plastique ( 2 ) avec un soufflet de chaque côté, se jette avec la protection absorbante après usage ( = propreté, hygiène, discrétion ).

La protection ( 1 ) et la housse plastique ( 2 ) se glissent dans la poche tissu ( 3 ), cf. figure 7-8 , Celle-ci comprend deux plis d'aisance. La poche en tissu est cousue au short, bermuda ou caleçon.

A noter que le fourreau en matière absorbante, peut également être plastifié à l'extérieur, afin d'assurer une double protection aux personnes Souffrant de très fortes incontinences urinaires et de ce fait, de les rassurer complètement.

## Revendications

1. Système de protection hygiénique pour incontinance urinaire masculine, impliquant une pièce de matière absorbante disposée dans une pièce de sous-vêtement destinée à être portée au niveau de l'organe urinaire masculin, **caractérisé en ce que** le système de protection implique
des pochettes en matière imperméable et
des pochettes ou fourreaux en matière absorbante, se glissant dans lesdites pochettes imperméables, ainsi qu'
un caleçon en matière textile quelconque, muni sur sa face interne au niveau de l'organe urinaire, d'une poche en matière textile quelconque, ladite poche en matière textile etant destinée à recevoir une desdites pochettes en matière imperméable munie d'une desdites pochettes ou fourreaux en matière absorbante.

2. Système de protection selon la revendication 1, **caractérisé en ce que** les pochettes en matière imperméable et des pochettes ou fourreaux en matière absorbante sont de type jetable.

3. Système de protection selon l'une ou l'autre des revendications précédentes, **caractérisé en ce que** le caleçon et la pochette interne sont constués d'une matière textile identique ou permettant un même traitement au lavage.

4. Système de protection selon l'une ou l'autre des revendications précédentes, **caractérisé en ce que** l'ouverture de la poche disposée sur la face interne du caleçon est située à environ 20 à 25 cm, de préférence à environ 22 cm, de la taille d'un homme standard,

5. Système de protection selon l'une ou l'autre des revendications précédentes, **caractérisé en ce que** la poche disposée sur la face interne du caleçon est située du coté gauche de la jambe, légèrement en oblique.

6. Caleçon en matière textile quelconque **caractérisé en ce qu'**il est muni sur sa face interne au niveau de l'organe urinaire, d'une poche en matière textile quelconque.

7. Caleçon selon la revendication 6, **caractérisé en ce que** l'ouverture de la poche disposée sur la face interne du caleçon est située à environ 20 à 25 cm, de préférence à environ 22 cm, de la taille d'un homme standard.

8. Caleçon selon l'une ou l'autre des revendication 6 et 7, **caractérisé en ce que** la poche disposée sur la face interne du caleçon est située du coté gauche de la jambe, légèrement en oblique.

9. Ensemble d'une pochette en matière imperméable et d'une pochette ou fourreau en matière absorbante, **caractérisé en ce qu'**il est destiné à être utilisé dans le système selon les revendications précédentes.
